# FASCICULE DE BREVET EUROPEEN

(11) **EP 0 531 263 B1**
(45) Date de publication et mention de la délivrance du brevet: **29.05.1996**
(21) Numéro de dépôt: 92830439.3
(22) Date de dépôt: 06.08.1992
(51) Int. Cl.: A61F 2/38

(54) **Prothèse pour genou équipée d'une articulation entre les composantes fémorales et tibiales et leurs respectifs pivots endomédullaires**
Knieprothese mit einem Gelenk zwischen den femoralen und tibialen Komponenten und den entsprechenden endomodularen Schäften
Knee prosthesis provided with articulations between femoral and tibial components and their respective stems

(30) Priorité: 08.08.1991 IT AN910031
(43) Date de publication de la demande: 10.03.1993
(73) Titulaire: GEMED LTD, Guernsey, Channel Islands (GB)
(72) Inventeur: Jacchia, Gian Eugenio, Firenze (IT)
(74) Mandataire: Baldi, Claudio

(56) Documents cités:
- EP-A- 0 376 658
- US-A- 3 916 451
- US-A- 4 822 366
- US-A- 4 985 037

## Description

La présente demande de brevet pour invention industrielle concerne une prothèse pour genou équipée d'une articulation des composantes fémorales et tibiales avec leurs respectives tiges médullaires; ladite articulation étant obtenue au moyen d'une vis dont la tête est en forme de calotte sphérique et qui relie lesdites composantes au pivot endomédullaire.

Les prothèses pour genoux sont normalement formées d'une composante fémorale, d'une composante tibiale, chacune desquelles étant équipée de son propre pivot ou tige médullaire à ficher, respectivement, dans le canal fémoral et dans le canal tibial.

Lesdits pivots résultent reliés à la composante fémorale et à la composante tibiale par le biais de moyens qui en permettent un facile et rapide démontage, de manière à pouvoir ainsi disposer d'éléments entre eux interchangeables, qui permettent d'assembler, tour à tour, une prothèse de forme et dimensions telles à s'adapter à la structure osseuse du patient. Une prothèse de ce type est celle illustrée dans le brevet US-4822366, qui décrit une prothèse à modules pour genou, apte au remplacement d'une partie de l'articulation du genou. L'ensemble est formé d'une composante fémorale réalisée de manière à inclure des premières surfaces de support et des premiers moyens aptes à recevoir - afin qu'il soit démontable - un élément à tige fémorale en option. L'ensemble est également formé d'une composante tibiale réalisée de manière à inclure une plate-forme et des seconds moyens aptes ä recevoir - afin qu'il soit démontable - un élément à tige tibiale en option.

L'ensemble inclut une insertion tibiale séparée, configurée de manière à être supportée par la plate-forme de la composante tibiale. L'insertion tibiale est réalisée de manière à inclure des secondes surfaces de support, configurées de manière à s'accoster avec les premières surfaces prévues sur la composante fémorale, afin de permettre le mouvement de pivotement entre la composante fémorale et la composante tibiale.

Dans les prothèses du type connu, les pivots, bien qu'étant interchangeables, sont fixés à la composante tibiale et à la composante fémorale de manière à éliminer toute possibilité d'articulation entre les parties reliées.

Le but de la présente invention est celui de concevoir une prothèse prévoyant une articulation entre les composantes fémorales et tibiales et leurs respectives tiges médullaires.

La prothèse selon l'idée offre simultanément les avantages des prothèses totales traditionnelles ainsi que ceux des prothèses fixées au moyen du vissage d'un pivot dans le canal médullaire.

En fait, la susdite particulière articulation permet d'obtenir une excellente stabilisation primaire, ainsi qu'une optimale stabilisation secondaire du moment que les micro-mouvements, aussi bien dans le sens axial que tangentiel, sont complètement empêchés.

Contrairement aux implantations qui sont fixées par vissage, la prothèse en question, bien qu'ayant une fixation à pivot, offre en plus la possibilité d'effectuer les résections fémorales et tibiales avec des angles pré-établis, de manière à obtenir le correct balancement des ligaments.

Il faut encore souligner que la prothèse en question, justement à cause de cette originale articulation réalisée entre les composantes fémorales et tibiales et leurs respectifs pivots endomédullaires, garantit l'adhésion de l'os à la prothèse le long de l'axe du fémur et du tibia, même en cas de forte ostéoporose, sans devoir utiliser du ciment.

En définitive, la jonction articulée entre le pivot vissé et la prothèse fémorale ou tibiale effectue un rôle très valable dans tous les cas de déformation angulaire du genou présents avant la phase opératoire en restituant à l'articulation une géométrie anatomique normale, obtenue avec le positionnement idéal des éléments fonctionnels par rapport à l'axe mécanique du membre; la prothèse en question est donc en mesure d'effectuer la correction tant du genu-valgum, que du genu-varum, du genu-recurvum, ainsi que du genu-flessum.

Une spéciale version de construction de la prothèse en question est prévue; celle-ci permet de varier le point d'arrêt de la tête de la vis à l'interne de la composante fémorale et tibiale, de manière à ce que le chirurgien orthopédiste puisse, dans toutes les situations, s'approcher le plus près possible aux conditions optimales de coaxialité entre le pivot et sa vis de fixation.

Dans cette perspective, le siège de logement, en forme de calotte sphérique, pour la vis qui fixe le pivot à la composante tibiale de la prothèse, a été réalisé dans un petit disque interchangeable que l'on positionne dans une cavité adaptée qui sera réalisée sur le plat tibial.

Une nombreuse série desdits petits disques interchangeables est prévue, chacun caractérisé par le placement plus ou moins excentrique du siège susdit; la simple substitution des susdits petits disques dans le plat tibial permet de déplacer à volonté l'axe de la vis, en le rendant ainsi le plus coaxial possible par rapport à l'axe du sous-jacent pivot fiché dans le canal médullaire du tibia.

De même sur la composante fémorale une fente a été réalisée afin de permettre à la vis de fixation du pivot fémoral de glisser en avant ou en arrière.

Ces avantages, ainsi que d'autres, de la prothèse en question seront plus évidents lors de la lecture de la description qui pour une meilleure explication se poursuit en faisant référence aux tableaux de dessin annexés qui ont valeur d'illustration et ne sont pas limitatifs et où:
- la fig. 1 montre la prothèse selon l'invention vue de côte et sectionnée sur un plan vertical médian;
- la fig. 2 montre la prothèse selon l'invention vue de front et partiellement sectionnée sur un plan vertical passant par l'axe du pivot tibial;
- la fig. 3 montre de manière schématique une version différente de construction du point d'articulation entre le pivot et la composante tibiale;
- la fig. 4 montre la composante fémorale de la prothèse dans la version spéciale, vue du haut;
- la fig. 5 montre la composante fémorale de la prothèse dans la version spéciale, vue du côté gauche, avec la vis de fixation du pivot enfilée dans la fente;
- la fig. 6 est une vue frontale de la composante fémorale de la prothèse dans la version spéciale, sectionnée avec le plan VI-VI de la fig. 2;
- la fig. 7 montre le plat tibial de la prothèse dans la version spéciale, vu du haut et équipé du petit disque interchangeable;
- la fig. 8 est la section avec le plan VIII-VIII de la fig. 7;
- la fig. 9 montre le plat tibial de la prothèse dans la version spéciale, vu du haut et privé du petit disque interchangeable;
- la fig. 10 est la section de la fig. 9 avec le plan X-X;
- la fig. 11 est la représentation d'une série de petits disques interchangeables.

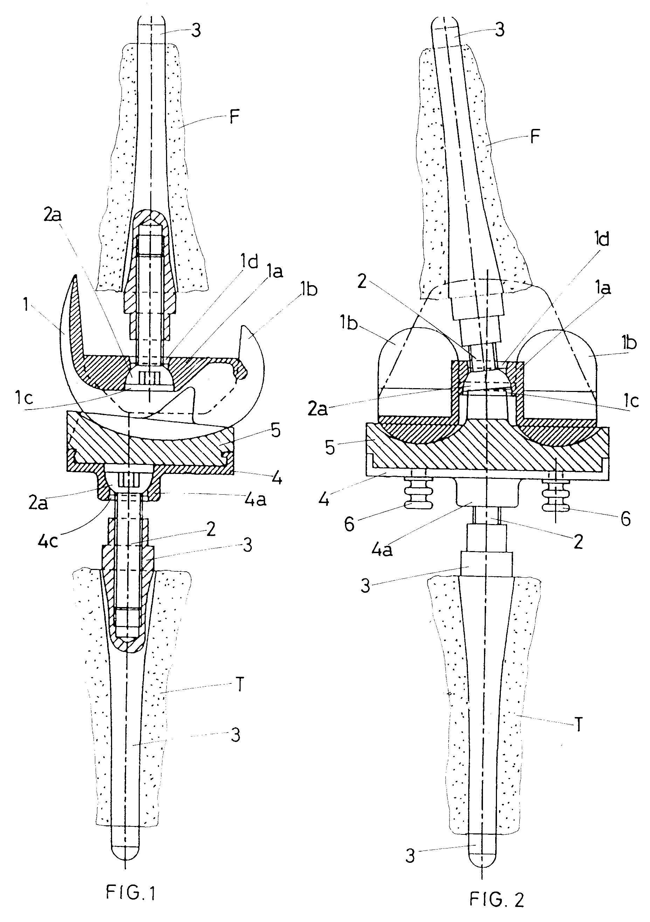

En Référence aux figures 1, 2 et 3, la prothèse en question englobe la prothèse fémorale (1), réalisée en métal et ayant une géométrie qui en permet l'implantation tant sur le genou de gauche que sur celui de droite; le pont central anti-rotation (1a) ainsi que les condyles fémoraux (1b) sont bien visibles dans la fig. 2.

L'union de la composante fémorale (1) au fémur (F) s'effectue au moyen d'une vis (2) que l'on visse de manière axiale dans un pivot (3) conventionnel, fiché dans la spongieuse fémorale.

Il faut prêter une attention particulière à la configuration de la tête (2a) de la vis de jonction (2) dont la tête est en forme de calotte sphérique et qui est nichée dans un siège (1c) conforme, creusé au centre du pont intercondylien (1a) de la composante fémorale (1).

Ledit siège (1c) présente sur son fond un perçage passant (1d) de diamètre plus grand que la tige filetée de la vis (2) qui peut donc assumer des positions avec différentes inclinations par rapport à l'axe du perçage (1d) susmentionné, comme mis en évidence dans la fig. 2.

Cette jonction articulée entre la tête (2a) de la vis (2) et son siège de logement (1c) comporte la réalisation de la désirée jonction articulée entre la composante fémorale (1) et son respectif pivot endomédullaire (3).

De même pour la composante tibiale, formée d'un plat métallique (4) fixé en-dessous d'une plate-forme (5), moulée en polyéthylène à forte densité, et dont la partie supérieure est formée d'une cavité moulée de manière à pouvoir se coupler avec les condyles fémoraux (1b).

En effet, le plat tibial (4) présente inférieurement une protubérance (4a) dans laquelle un siège (4b) est creusé; ledit siège ayant une forme sphérique et sur le fond un perçage passant (4c) de diamètre plus grand de la tige filetée de la vis (2) qui se visse de manière axiale dans le pivot (3) fiché dans le canal médullaire du tibia (T).

Il faut souligner que les pivots (3) présentent un filetage à pas large, adapté à la nature spongieuse de l'os, et une surface micro-poreuse apte à favoriser l'ancrage sans ciment.

Deux arrêts en forme d'anneau, anti-rotation et forjettant inférieurement du plat tibial (4) sont représentés dans la fig. 2 avec le numéro (6).

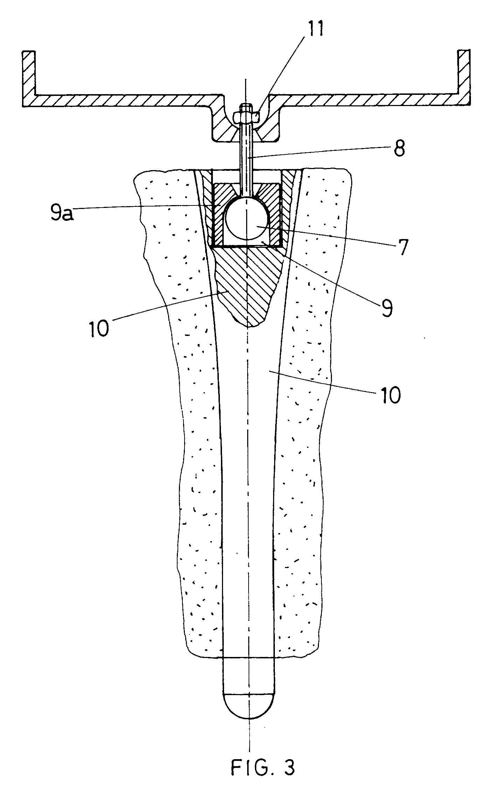

Toutes les composantes métalliques sont réalisées en alliage de titane ou dans un autre alliage biocompatible. On peut prévoir que le point d'articulation sphérique entre le pivot et la composante fémorale ou tibiale puisse être réalisé à l'interieur du pivot même, comme illustré de manière schématique dans la fig. 3, et où ladite forme de réalisation alternative de l'idée a été illustrée uniquement en faisant référence à la composante tibiale.

Dans ce cas la tête sphérique (7) de la vis de jonction (8) est nichée dans un siège conforme (9) percé et prévu dans un support adapté (9a) fixé à la base du pivot (10); la composante fémorale ou tibiale doit alors prévoir un siège adapté pour le logement de l'écrou de serrage (11) qui fixe la tige filetée de la susdite vis (8) à la composante même. Référence les figures de 4 à 11, examinons maintenant la configuration assumée par la prothèse en question dans la version spéciale, apte à permettre un réglage du point d'arrêt de la vis sur les composantes fémorale et tibiale.

La composante fémorale (12) dans cette version spéciale résulte privée du susdit pont intercondylien (1a) de manière à préserver le capital osseux.

Dans la version spéciale, la composante fémorale (12) supporte une fente centrale (13) ayant les bords (13a) arrondis et dans laquelle la tête (2a) de la vis (2) qui se visse sur le pivot fémoral a son exact logement.

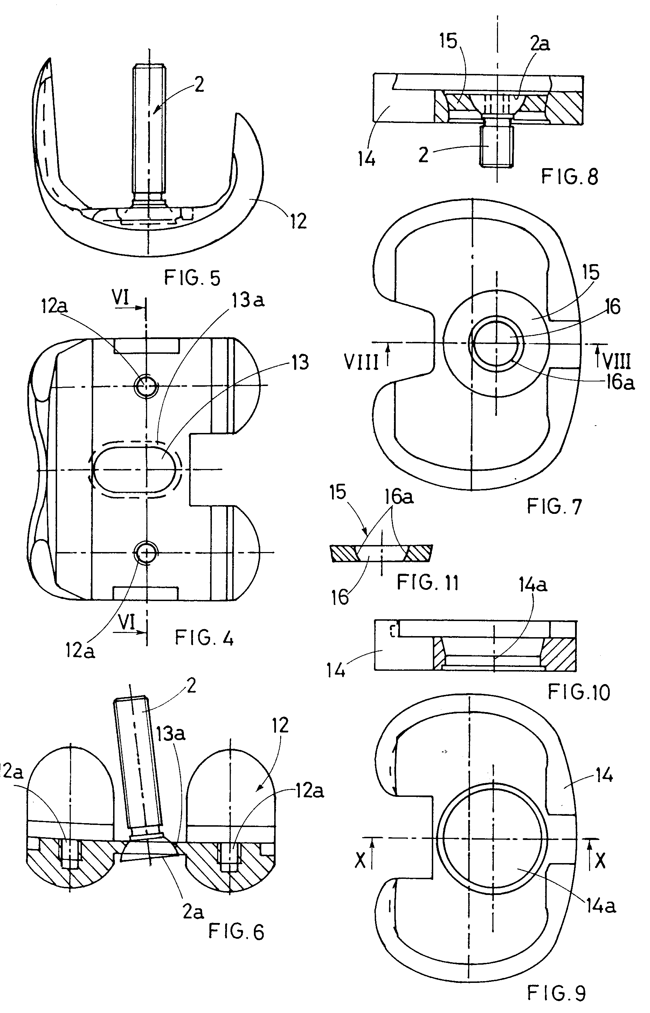

Ainsi que mis en évidence à la fig. 6, la largeur de la fente (13), ainsi que ses bords arrondis (13a) sont tels qu'ils permettent la liberté d'oscillation de la tête (2a) et par conséquent de la tige filetée de la vis (2), tandis que la longueur de la fente (13), clairement visible à la figure 4, permet les déplacements antéro-postérieurs de la vis (2).

Référence les figures de 7 à 11, le plat tibial (14) dans la version spéciale de la prothèse en question présente un perçage central (14a) avec embouchure tronconique dans laquelle un petit disque interchangeable (15) a son exact logement; sur ledit petit disque interchangeable (15), en position plus ou moins excentrique, un perçage (16) a été réalisé ayant des bords arrondis (16a) et dans lequel la tête (2a) de la vis (2) qui se visse dans le pivot tibial a son exact logement.

Le profil concave des bords (16a) des perçages (16) se conjugue parfaitement avec celui convexe de la tête (2a) de la vis (2), permettant ainsi à cette dernière des oscillations par rapport à l'axe du perçage (16).

On fait de même remarquer que les petits disques (15) étant en mesure de pivoter dans leurs propres logements (14a), la possibilité de réglage de la vis (2) est vraiment très vaste, vu qu'on peut l'effectuer soit en choisissant l'un des plusieurs petits disques disponibles, soit à travers une rotation adaptée du petit disque choisi dans son logement du plat tibial.

Il faut aussi souligner que la prothèse en question est en mesure d'utiliser, pour le raccordement et la fixation au canal endomédullaire, non seulement des pivots mais bien aussi des coins; lesdits coins devraient alors être équipés d'un perçage fileté conforme dans lequel pourrait s'engager la tige de la vis (2) qui dans ce cas présenterait des longueurs inférieures de celles utilisées avec les pivots. L'utilisation des susdits coins est parfois préférée à celle des pivots, surtout pour la fixation de la composante tibiale de la prothèse.

Finalement on souligne que dans la composante fémorale (12), deux perçages filetés (12a) sont prévus; en position médio-latérale et dans lesquels, si nécessaire, on peut visser des pivots que l'on veut ficher dans l'os fémoral, en renfort ou en alternative du pivot fémoral.

## Revendications

1. Prothèse pour genou équipée d'une articulation entre les composantes fémorales et tibiales et leurs respectifs pivots endomédullaires, du type comprenant une composante fémorale (1), un plat tibial (4), des pivots endomédullaires (3) et des moyens aptes à la fixation interchangeable des pivots (3) aux composantes fémorales et tibiales, caractérisée en ce que les pivots endomédullaires (3) sont reliés respectivement à la composante fémorale (1) et au plat tibial (4) au moyen d'une vis (2) dont la tête (2a) est en forme de calotte sphérique et nichée dans des sièges de forme complémentaire (1c et 4b) respectivement réalisés sur la composante fémorale (1) et sur le plat (4), et ayant au fond de la calotte un perçage passant (1d et 4c) dont le diamètre est plus grand que celui de la tige filetée de la vis (2).

2. Prothèse pour genou selon la revendication 1), caractérisée en ce que sa forme de réalisation alternative présente le fait que la tête sphérique (7) de la vis de jonction (8) soit logée dans un siège de forme complémentaire percé (9) et prévu dans un support adapté (9a) fixé a l'interieur à la base du pivot endomédullaire (10); étant prévu que la composante fémorale et tibiale présentent, dans cette version, un siège adapté pour le logement de l'écrou de serrage (11) qui fixe la tige filetée de la susdite vis (8) à la composante même.

3. Prothèse pour genou, selon la revendication 1), caractérisée en ce que les pivots (3) présentent un filetage à pas large et une surface micro-poreuse.

4. Prothèse pour genou, selon la revendication 1), caractérisée en ce que, le plat tibial (14) présente un perçage central (14a) ayant une embouchure tronconique dans laquelle un petit disque interchangeable (15) a son exact logement et sur lequel a été réalisé, en position plus ou moins excentrique, un perçage (16) ayant des bords arrondis (16a) et dans lequel la tête (2a) de la vis (2) vissée dans le pivot tibial a son exact logement.

5. Prothèse pour genou, selon la revendication 1), caractérisée en ce que, la composante fémorale (12) présente une fente centrale (13) ayant des bords arrondis (13a) et dans laquelle la tête (2a) de la vis (2) vissée dans le pivot fémoral a son exact logement, ayant ainsi la possibilité de déplacements antéro-postérieurs.

## Claims

1. A knee prosthesis with joints between the femoral and tibial components and the respective intramedullar stems (1), of the type having a femoral component (1), a tibial plate (4), intramedullar stems (3) and means for interchangeable fixing of the stems (3) to the femoral and tibial components characterised in that the intramedullar stems (3) are connected respectively to the femoral component (1) and to the tibial plate (4) by means of a ball-head (2a) screw (2) housed in conforming housings (1c and 4b) on component (1) and on plate (4) respectively, and having a through hole (1d and 4c) on the bottom, whose diameter is greater than the threaded shank of the screw (2).

2. A knee prosthesis according to claim 1, characterised in that the ball-head (7) of the connecting screw (8) is housed in a drilled housing (9) having conforming shape engineered in a support (9a) fixed internally at the base of the intramedullar stem (10); the femoral and tibial components being characterised in this embodiment by a housing for the nut (11) that fixes the threaded shank of screw (8) to the component.

3. A knee prosthesis according to claim 1), characterised in that the stems (3) have a wide-pitched threading and microporous surface.

4. A knee prosthesis according to claim 1), characterised in that the tibial plate (14) has a centre hole (14a) with a truncated-cone mouth in which an interchangeable disk (15) is housed, said disk (15) having a more or less eccentric hole (16) with rounded edges (16a) housing the head (2a) of the screw (2) fitted in the tibial stem.

5. A knee prosthesis according to claim 1) characterised in that the femoral component (12) has a centre slot (13) with rounded edges (13a) housing the head (2a) of the screw (2) fitted in the femoral stem, sliding backwards and forwards.

## Patentansprüche

1. Knieprothese mit Gelenk zwischen den Oberschenkel- und Schienbeinkomponenten und entsprechenden Endomarkschäften, bestehend aus einer Oberschenkelkomponente (1), einer Schienbeinplatte (4), Endomarkschäften (3) und austauschbaren Befestigungsmitteln zur Befestigung der Schäfte (3) an den Oberschenkel- und Schienbeinkomponenten, dadurch gekennzeichnet, daß die Endomarkschäfte (3) je an die Oberschenkelkomponente (1) und an die Schienbeinplatte (4) mittels einer Schraube (2) befestigt werden, welche einen kalottenförmigen Kopf (2a) aufweist, der in entsprechenden Sitzen (1c und 4b) aufgenommen wird, die jeweils an der Komponente (1) und an der Platte (4) ausgebildet sind und am Boden eine Durchgangsöffnung (1d und 4c) mit einem Durchmesser besitzen, der größer ist, als der Gewindeschaft der Schraube (2).

2. Knieprothese, gemäß Patentanspruch 1) dadurch gekennzeichnet, daß der kugelförmige Kopf (7) der Verbindungsschraube (8) in einem gebohrten Sitz (9) liegt, der eine entsprechende Form aufweist und in einer geeigneten Halterung (9a) eingearbeitet ist, die innen an der Basis des Endomarkschafts (10) befestigt ist, wobei die Oberschenkel- und Schienbeinkomponenten in dieser Ausführung einen geeigneten Sitz für die Befestigungsmutter (11), welche den Gewindeschaft der Schraube (8) an derselben Komponente befestigt, vorweisen.

3. Knieprothese, gemäß Patentanspruch 1) dadurch gekennzeichnet, daß die Schäfte (3) ein Gewinde mit hoher Steigung und eine mikroporöse Oberfläche aufweisen.

4. Knieprothese, gemäß Patentanspruch 1) dadurch gekennzeichnet, daß die Schienbeinplatte (14) eine zentrale Öffnung (14a) mit einem kegelstumpfförmigen Eingang besitzt, in den eine austauschbare Scheibe (15) präzise hineinpaßt, auf der sich in einer mehr oder weniger exzentrischen Position eine Öffnung (16) mit abgerundeten Rändern (16a) befindet, in die der Kopf (2a) der Schraube (2), welche in einen Schienbeinschaft eingeschraubt ist, aufgenommen wird.

5. Knieprothese, gemäß Patentanspruch 1) dadurch gekennzeichnet, daß die Oberschenkelkomponente (12) einen zentralen Schlitz (13) mit abgerundeten Rändern (13a) aufweist, in den sich der Kopf (2a) der in den Oberschenkelschaft hineingeschraubten Schraube (2) präzise einpaßt, wobei besagte Schraube vor- und rückwärts gleiten kann.
